# EUROPEAN PATENT APPLICATION

(11) **EP 2 039 286 A1**
(43) Date of publication of application: **25.03.2009**
(21) Application number: 07018570.7
(22) Date of filing: 21.09.2007
(51) Int. Cl.: A61B 5/00

(54) **Interface device between data management unit and medical device**

(71) Applicant: Roche Diagnostics GmbH, 68305 Mannheim (DE); F.HOFFMANN-LA ROCHE AG, 4070 Basel (CH)
(72) Inventor: Hampel, Ferdinand, 68163 Mannheim (DE)

(57) **Abstract**

The present invention refers to a system comprising a portable data management unit (DMU) and an interface device (ID) for transferring data from a medical device (MD) to the DMU or from the DMU to the MD, the ID comprising a microprocessor programmed to enable data conversion and data transfer between the MD and the DMU, an energy source, a MD-ID transceiver to wirelessly receive data from the MD as an electromagnetic signal or to wirelessly transmit data to the MD as an electromagnetic signal, an ID-DMU transceiver to wirelessly transmit data to the DMU as an electromagnetic signal, or to wirelessly receive data from the DMU as an electromagnetic signal, wherein the data transferred as electromagnetic signals between the MD and the ID are transmitted by executing a first transmission protocol, and the data transferred as electromagnetic signals between the ID and the DMU are transmitted by executing a second transmission protocol, the first and second transmission protocols being different.

## Description

### Field of the invention

The invention relates to an interface device (ID) for the wireless transfer of data between a medical device (MD) and a portable data management unit (DMU) such as a personal digital assistant (PDA) or a Smartphone. The medical device is a device for measuring analytes in a body fluid or a body fluid parameter or for administration of therapeutic agents. The MD is also portable.

### Background

In many fields of healthcare, repeated measurement and monitoring of certain analytes present in body fluids, such as blood or urine, is of particular importance. One special case concerns for example patients affected by diabetes who need to measure very frequently the concentration of glucose in order to respond promptly with the correct medication. If certain limits are indeed exceeded, the affected patient might even incur in coma and death. The high concentration of glucose in the blood is also responsible for well known side effects gradually deteriorating the state of health. Therefore long term monitoring is also required in order to keep the glycemic level under control. Blood glucose data are thus useful both to the physician who has the task to determine the most appropriate long-term therapy and to the patient who daily needs to adapt the administration of medications according to the measured glucose levels. These depend in fact not only on the diet, but also on the daily physical activity and many other factors, which influence the metabolism.

A number of small, reliable and low-cost medical devices, which can be handheld, are today available to the patient for self monitoring. Devices for controlled administration of therapeutic agents, such as insulin pumps, are also commercially available.

The number of examples of medical devices to which this invention refers to is however not limited to diabetes care. Worth mentioning are for example also those devices for monitoring blood pressure or other blood parameters like the coagulation factors.

In parallel, handheld data processing and management units are also becoming of widespread use. Examples are small portable computers, PDAs, mobile phones and Smartphones, a sort of PDA which also includes a mobile telephone function. These, in most cases, incorporate interface ports for transferring or exchanging data between different devices. This sort of communication occurs most conveniently wirelessly. Widely used is for example a kind of short-range free-space optical communication based on infrared light. Often used in this regard is a standard IrDA (Infrared Data Association) protocol. The wavelength chosen for this standard is between 850 nm and 900 nm.
Another convenient wireless communication is based on short-range radio frequency (2.4 GHz), using a standard protocol referred to as Bluetooth.

A protocol is a convention or standard that controls or enables the connection, communication, and data transfer between two devices. In its simplest form, a protocol can be defined as the rules governing the syntax, semantics, and synchronization of communication. These rules comprise data representation, signaling, authentication and error detection required to transmit data. Protocols may be implemented by hardware, software, or a combination of the two.

Among the common advantages of both IrDA and Bluetooth are reliability, safety of transmission and the fact that they use small and cheap transmitters/receivers or transceivers.
Importantly, the data transmission/reception can be performed with low power consumption.

It is an object of the present invention to facilitate the communication between data management units and medical devices.

Using an external DMU is often more convenient than incorporating this function into a MD. The reasons are mainly related to development speed and costs of a MD, reflected on the MD price, but also to the ease of use of a MD and the fact that DMUs are already available. DMUs can be indeed efficiently used e.g. for displaying, storing, comparing, plotting, averaging, and in turn eventually transferring the information to a server or to a doctor. They might be even used to control the operation of the medical device by executing a specific and defined instruction in time or in response to the data acquired from the same or another medical device. Monitoring of the state of health and optimization of treatments for individuals in this way can be improved.

One problem in the state of the art is that communication protocols between medical devices and data management units are often not the same, thus raising a compatibility issue. Specifically, while a data management unit typically uses a standard protocol such as IrDA or Bluetooth, the medical devices typically use a non standard proprietary protocol. One group of these devices is for example that belonging to the Accu-Check^{®} family of products from Roche Diagnostics, which communicate by means of a non standard infrared protocol.

This protocol can be made available to personal computers and PDAs by installing software and drivers. An interface adapter is however preferred because no installation is necessary. One such adapter is already available, the so called Accu-Check Smart Pix^{®}. This device acts as an interface for the various Accu-Check^{®} meters and insulin pumps by means of an infrared channel, and it also contains the electronics and the programs for processing the data input and measurements. The measured data are stored in the device temporarily and can be viewed with an Internet browser after being transferred to a computer via a standard USB (Universal Serial Bus) cable. A display on the interface indicates the operating status. For medical devices, which do not have a built-in infrared port, an IR-Key, which is pluggable into the MD, is also available in order to allow infrared data communication with the interface device. The Accu-Check Smart Pix^{®} interface however does not allow direct wireless communication with DMUs but only USB cable communication with data processing units such as computers, which comprise a USB port. Handheld DMUs are often not equipped with a USB port.

It is thereby an object of the present invention to provide a new and more convenient interface device allowing communication between a portable MD using a wireless non standard communication protocol and a portable DMU using another communication protocol,
wherein the ID is small, portable, inexpensive and works with low power consumption.

### Description of the invention

The present invention refers to a system comprising a portable DMU and an ID for transferring data from a MD to the DMU or from the DMU to the MD, the ID comprising a microprocessor programmed to enable data conversion and data transfer between the MD and the DMU, an energy source, a MD-ID transceiver to wirelessly receive data from the MD as an electromagnetic signal or to wirelessly transmit data to the MD as an electromagnetic signal, an ID-DMU transceiver to wirelessly transmit data to the DMU as an electromagnetic signal, or to wirelessly receive data from the DMU as an electromagnetic signal, wherein the data transferred as electromagnetic signals between the MD and the ID are transmitted by executing a first transmission protocol, and the data transferred as electromagnetic signals between the ID and the DMU are transmitted by executing a second transmission protocol, the first and second transmission protocols being different.

According to another embodiment the system comprises also the MD.

A MD according to the present invention is preferably a handheld device, operated by the individual patient for self testing and monitoring. The tests may concern the concentration of analytes, in a body fluid such as blood or urine, or a blood parameter such as blood pressure. Examples are glucose meters, coagulation factor meters, blood pressure meters. Another class of devices however concerns the controlled administration of therapeutic agents. Examples are insulin pumps or insulin pens. A MD may not only record or report the concentration value of a measured analyte, but other information too such as the time of the measurement, a batch number, the dose and time of a therapeutic agent delivered, e.g. insulin data, the serial number of the MD, etc. All of which are transmittable data.
A MD comprises either an analytical test section or a drug administration section or both. The analytical test section may comprise for example a port to accommodate an analytical test strip and an optical or electrochemical detector. The drug administration section may comprise a pump and an infusion catheter.

A portable DMU according to the present invention is preferably a multifunctional electronic unit comprising hardware such as a microprocessor, a memory, a display and interface ports, as well as software or programs, for processing, storing, displaying personal documents or data, and for communicating preferably wirelessly or via wire with similar or other devices. Examples are small portable computers, personal digital assistants (PDAs), mobile phones and Smartphones, where mobile telephone, camera, and/or a global positioning system (GPS) functions may be also integrated.

The present invention provides an ID, which enables communication between a MD and a DMU when the protocols that they use are not the same.

The ID comprises a microprocessor and programs for elaboration and/or conversion of data in e.g. a format that can be easily displayed by standard software or browser running on the DMU. For this purpose the ID may comprise a memory for temporary data storage. But the main task of the microprocessor is to convey data transmitted as electromagnetic signals either from the MD or the DMU into another electromagnetic signal which can be recognized and interpreted either by the DMU or by the MD.

The ID comprises also an energy source, which can be for example a battery, thus eliminating the need of cables as power supply. The ID comprises also transceivers or individual transmitters and receivers for the wireless transmission/reception of electromagnetic signals.
Examples of such electromagnetic signals are infrared and radiofrequency signals.

In a preferred embodiment according to the present invention the electromagnetic signals transmitted between the MD and the ID are infrared (IR) signals transmitted by executing a non standard IR transmission protocol. Non standard means a proprietary protocol associated with the MD by the manufacturer of the MD, and not commonly associated with other devices such as DMUs.

In a preferred embodiment a MD comprises an integrated transceiver for the direct wireless transmission/reception of data as electromagnetic signals. It could be that a MD comprises no integrated transceiver and is unable to transmit or receive data directly. In this case the MD could comprise however a pluggable module or key, e.g. an IR module, which would give the MD the possibility to exchange data with the ID, thus acting as further interface between the MD and the ID.

The electromagnetic signals transmitted between the ID and the DMU are typically signals transmitted by executing a standard transmission protocol, where standard means a protocol commonly associated with devices of widespread use such as DMUs, often without legal restrictions.

According to one embodiment the electromagnetic signals transmitted between the ID and the DMU are for example infrared signals transmitted by executing a standard IR transmission protocol, e.g. a standard IrDA protocol.

According to another embodiment the electromagnetic signals transmitted between the ID and the DMU are for example radio frequency (RF) signals transmitted by a standard RF transmission protocol, e.g. a Bluetooth protocol.

The ID preferably comprises both an IrDA transceiver and a RF transceiver, and executes respective protocols. Different and/or additional wireless standard and non standard protocols other than those based on infrared light or radiofrequency could be also used.

The ID may be able to communicate in addition also with a stationary data management unit (SDMU), e.g. a desktop PC. Such a communication may be wireless, e.g. using IrDA or Bluetooth protocols, or wire-based, using e.g. USB ports.

The present invention further discloses a method of wirelessly transferring data from a MD to a portable DMU or from a DMU to a MD by means of an ID, characterized in that the ID receives data as electromagnetic signals from the MD or from the DMU by means of at least one transceiver, the ID converts the received data by means of a microprocessor, the ID transmits the converted data as electromagnetic signals to the DMU or to the MD by means of at least one transceiver, wherein the data transferred between the MD and the ID are transmitted by executing a first transmission protocol, and the data transferred between the ID and the DMU are transmitted by executing a second transmission protocol, the first and second transmission protocols being different.

According to one method the data between the MD and the ID are transferred as infrared electromagnetic signals by executing a non standard transmission protocol, while the data between the ID and the DMU may be transferred as infrared electromagnetic signals by executing a standard IR transmission protocol, e.g. a standard IrDA protocol, or as radio frequency electromagnetic signals by executing a standard RF transmission protocol, e.g. a Bluetooth protocol. Which transmission protocol is chosen, depends on the DMU compatibility.
The ID preferably performs a port scan to check for the presence of enabled communication ports on the DMU which are compatible. When more than one of these ports is detected, one of these could be automatically selected. An accordingly automatically selected protocol will then initiate and data transfer begins. The ID could also offer a switcher for choosing the transmission path. When more than one enabled port is detected, the user might be asked to choose one.

In alternative, a wire-based connection could be established if the DMU comprises a USB port too, e.g. with a desktop PC.

According to one method the ID is used to transfer data from a MD to a DMU and the data transferred from the MD to the DMU concern the concentration of analytes in a body fluid or a body fluid parameter and any extra information eventually recorded together with the measurement.

According to another method the ID is used to transfer data or instructions from a DMU to a MD, e.g. to control the administration of a therapeutic agent.

According to another method the ID converts the data received from the MD so that the data transferred to the DMU are in a format that can be displayed by the DMU with standard software or browser, e.g. a standard internet browser.

More in detail, the present invention can be best understood when read in conjunction with the following drawings, representing favorite embodiments, in which:
Figure 1 represents schematically some possible ways of transferring data wirelessly from a medical device (MD) to a portable data management unit (DMU) by means of an interface device (ID).
Figure 2 represents schematically some possible ways of transferring data wirelessly from a portable data management unit (DMU) to a medical device (MD) by means of an interface device (ID).
Figure 3 represents schematically the system of figure 1 with the addition of a further possible wire-based communication between the interface device (ID) and a stationary data management unit (SDMU).

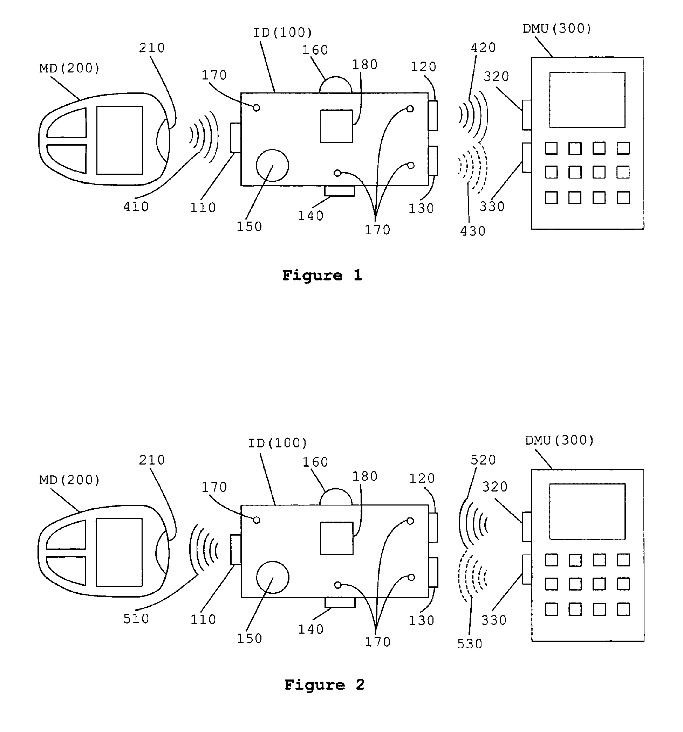

In figure 1 a system comprising a handheld DMU 300 and an ID 100 for transferring data from a MD 200 to the DMU 300 is schematically shown. The MD 200 comprises a transceiver 210 to transmit data to the ID 100 as a first electromagnetic signal 410. The ID 100 comprises a microprocessor 180 programmed to enable data conversion and data transfer between the MD 200 and the DMU 300, an energy source 150, one MD-ID transceiver 110 to receive data as a first electromagnetic signal 410 from the transceiver 210 of the MD 200 and one ID-DMU transceiver 120 to transmit data as a second electromagnetic signal 420 to the DMU 300. The interface device 100 may comprise also one or more alternative ID-DMU transceivers such as transceiver 130 to transmit data as an alternative second electromagnetic signal 430 to the DMU 300. The ID device 100 may in addition comprise also a port 140, preferably a USB port.
The DMU 300 comprises a transceiver 320 to receive data as a second electromagnetic signal 420 from the transceiver 120 of the ID 100. The DMU 300 may comprise also one or more alternative transceivers such as transceiver 330 to receive data as an alternative second electromagnetic signal 430 from the transceiver 130 of the ID 100.
Data are transmitted wirelessly as a first electromagnetic signal 410 between the MD 200 and the ID 100 by executing a first transmission protocol. Data are then converted by the microprocessor 180 and transmitted wireless as a second electromagnetic signal 420 or 430 to the DMU 300 by executing a second transmission protocol, the first and second transmission protocols being different.

In one favorite embodiment according to the present invention, the electromagnetic signal 410 transmitted between transceiver 210 of the MD 200 and the MD-ID transceiver 110 of the ID 100 is preferably an infrared signal transmitted by executing a non standard IR transmission protocol.
The electromagnetic signal 420 transmitted between the ID-DMU transceiver 120 of the ID 100 and the transceiver 320 of the DMU 300 is preferably an infrared signal transmitted by executing a standard IR transmission protocol, e.g. a standard IrDA protocol. Alternatively, the electromagnetic signal 430 transmitted between the ID-DMU transceiver 130 of the ID 100 and the transceiver 330 of the DMU 300 may be a radio frequency signal transmitted by executing a standard RF transmission protocol, e.g. a Bluetooth protocol.

In figure 2, according to another favorite embodiment, data are transmitted wirelessly via the ID 100 from the DMU 300 to the MD 200. One possibility is to transfer data as an electromagnetic signal 520 from the transceiver 320 of the DMU 300 to the ID-DMU transceiver 120 of the ID 100, where the electromagnetic signal 520 is for example an infrared signal transmitted by executing a standard IR transmission protocol, e.g. a standard IrDA protocol. Another possibility is to transfer data as an electromagnetic signal 530 from the transceiver 330 of the DMU 300 to the transceiver ID-DMU 130 of the ID 100, where the electromagnetic signal 530 is for example a radiofrequency signal transmitted by executing a standard RF transmission protocol, e.g. a standard Bluetooth protocol. In any case, data are then converted by the microprocessor 180 and transmitted wireless as an electromagnetic signal 510 from the MD-ID transceiver 110 of the ID 100 to the transceiver 210 of the MD 200 where the electromagnetic signal 510 is for example an infrared signal transmitted by executing a non standard IR transmission protocol.

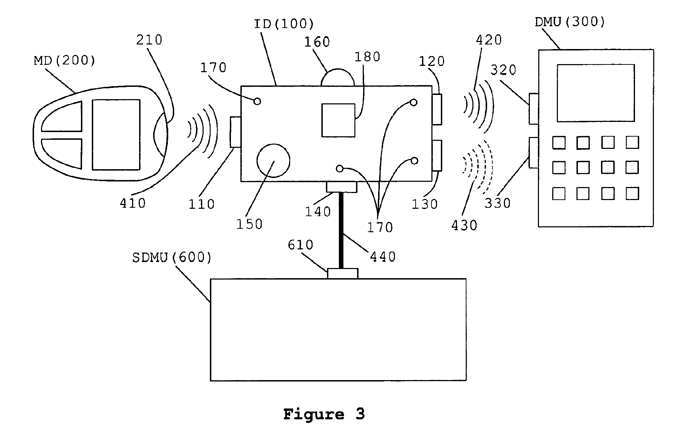

In figure 3, the same system of figure 1 is shown. According to one embodiment, port 140 of the ID 100 allows however a further optional connection via wire 440 between the ID 100 and a SDMU 600 comprising a port 610. According to one favorite embodiment the ports 140 and 610 are USB ports and the SDMU 600 is a desktop PC. Of course, the SDMU 600 may be able to communicate with the ID 100 also wirelessly. Analogously also a portable DMU 300 may be able to communicate with the ID 100 via wire 440 if said DMU 300 further comprises a USB port.
The ID 100 preferably comprises also a display (not shown) or a series of LED (Light-Emitting Diodes) 170, one in correspondence of each transceiver to indicate the operating status. An "on" status could be indicating for example that a connection has been established via the corresponding protocol. A blinking status could be indicating for example that data transfer is taking place in that moment.

The ID 100 could also conveniently comprise memory modules (not shown) for temporarily storing received data and converted data before being transmitted, while a simple switch button 160 could turn the power of the ID 100 on or off depending on use or initiate a protocol scan.

The ID 100 may comprise a switcher (not shown) to select one of the available transceivers 120, 130 or USB port 140 for data transmission between ID 100 and DMU 300 or SDMU 600.

Of course instead of two transceivers 110 and 120, as here represented for ease of understanding, only one could be used for receiving/transmitting IR electromagnetic signals, while two different protocols would be executed for communicating with the MD 200 and the DMU 300 respectively.

Also, instead of transceivers with the double function of receiver and transmitter, individual receivers and individual transmitters could be used.

## Claims

1. System comprising a portable data management unit (DMU) and an interface device (ID) for transferring data from a medical device (MD) to the DMU or from the DMU to the MD, the ID comprising
- a microprocessor (180) programmed to enable data conversion and data transfer between the MD and the DMU,
- an energy source (150),
- a MD-ID transceiver (110) to wirelessly receive data from the MD as an electromagnetic signal (410) or to wirelessly transmit data to the MD as an electromagnetic signal (510),
- at least one ID-DMU transceiver (120 and/or 130) to wirelessly transmit data to the DMU as an electromagnetic signal (420 or 430), or to wirelessly receive data from the DMU as an electromagnetic signal (520 or 530),
wherein
the data transferred between the MD and the ID are transmitted by executing a first transmission protocol,
and the data transferred between the ID and the DMU are transmitted by executing a second transmission protocol, the first and second transmission protocols being different.

2. The system of claim 1 further comprising the MD.

3. System according to claim 1 or 2, **characterized in that** the electromagnetic signals transmitted between the MD and the ID are infrared (IR) signals transmitted by executing a non standard IR transmission protocol.

4. System according to claim 3, **characterized in that** the MD comprises a pluggable IR module.

5. System according to any of the claims 1 to 4, **characterized in that** the electromagnetic signals transmitted between the ID and the DMU are infrared signals transmitted by executing a standard IR transmission protocol, e.g. a standard IrDA protocol.

6. System according to any of the claims 1 to 4, **characterized in that** the electromagnetic signals transmitted between the ID and the DMU are radio frequency (RF) signals transmitted by a standard RF transmission protocol, e.g. a Bluetooth protocol.

7. System according to any of the preceding claims, **characterized in that** the ID comprises an IrDA transceiver and a RF transceiver.

8. System according to any of the preceding claims, **characterized in that** the ID further comprises a port, preferably a USB port, for transmitting data via wire between the ID and a stationary data management unit, e.g. a desktop PC.

9. System according to claim 2, **characterized in that** the MD is a device for measuring or monitoring the concentration of analytes in a body fluid or a body fluid parameter.

10. System according to claim 2, **characterized in that** the MD is a device for administration of therapeutic agents.

11. System according to any of the preceding claims, **characterized in that** the DMU is a portable computer, a PDA, a mobile phone or a Smartphone.

12. System according to any of the preceding claims, **characterized in that** the energy source is a battery.

13. Method of wirelessly transferring data from a medical device (MD) to a portable data management unit (DMU) or from a DMU to a MD by means of an interface device (ID), **characterized in that**
- the ID comprises an energy source,
- the ID receives data as electromagnetic signals from the MD or from the DMU by means of at least one transceiver,
- the ID converts the received data by means of a microprocessor,
- the ID transmits the converted data as electromagnetic signals to the DMU or to the MD by means of at least one transceiver,
wherein
the data transferred between the MD and the ID are transmitted by executing a first transmission protocol,
and the data transferred between the ID and the DMU are transmitted by executing a second transmission protocol, the first and second transmission protocols being different.

14. Method according to claim 13, **characterized in that** data between the MD and the ID are transferred as infrared electromagnetic signals by executing a non standard transmission protocol.

15. Method according to claim 13 or 14, **characterized in that** data between the ID and the DMU are transferred as infrared electromagnetic signals by executing a standard IR transmission protocol, e.g. a standard IrDA protocol.

16. Method according to claim 13 or 14, **characterized in that** data between the ID and the DMU are transferred as radio frequency electromagnetic signals by executing a standard RF transmission protocol, e.g. a Bluetooth protocol.

17. Method according to claims 15 and 16, **characterized in that** data between the ID and the DMU are transferred either as an IR electromagnetic signal or as a RF electromagnetic signal depending on the DMU compatibility.

18. Method according to any of the claims 13 to 17, **characterized in that** the data transferred from a MD to a DMU concern the concentration of analytes in a body fluid or a body fluid parameter.

19. Method according to any of the claims 13 to 18, **characterized in that** data are transferred from a DMU to a MD to control the administration of a therapeutic agent.

20. Method according to any of the claims 13 to 19, **characterized in that** the data transferred from the MD to the DMU are in a format that can be displayed by the DMU with standard software or browser.
